(19) Europäisches Patentamt
European Patent Office
Office européen des brevets

(11) **EP 1 958 150 B1**

(12) **EUROPEAN PATENT SPECIFICATION**

(45) Date of publication and mention
of the grant of the patent:
**22.05.2013  Bulletin 2013/21**

(51) Int Cl.:
*G06T 5/00* (2006.01)          *G06T 7/00* (2006.01)
*A61B 6/00* (2006.01)          *A61B 5/103* (2006.01)
*G01B 11/245* (2006.01)

(21) Application number: **06804670.5**

(22) Date of filing: **01.11.2006**

(86) International application number:
**PCT/CA2006/001795**

(87) International publication number:
**WO 2007/051299 (10.05.2007 Gazette 2007/19)**

(54) **SURFACE ANALYSIS METHOD AND SYSTEM**

VERFAHREN UND SYSTEM ZUR OBERFLÄCHENANALYSE

PROCEDE ET SYSTEME D'ANALYSE DE SURFACES

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **04.11.2005  US 733178 P**

(43) Date of publication of application:
**20.08.2008  Bulletin 2008/34**

(73) Proprietor: **Cryos Technology, Inc.
Québec J6E 1T7 (CA)**

(72) Inventor: **PERRAULT, Ronald
deceased (CA)**

(74) Representative: **Patentanwaltskanzlei
Matschnig & Forsthuber OG et al
Siebensterngasse 54
1071 Wien (AT)**

(56) References cited:
WO-A2-2005/065293      US-A- 4 519 041
US-A- 5 682 443        US-B1- 6 697 516
US-B1- 6 697 516

• **Élodie Touchette: "Identification of the
morphological parameters characteristic of pes
cavus and the development of a prediction
model", , 1 June 2003 (2003-06-01), XP55004607,
Retrieved from the Internet: URL:http:
//www.cryos.com/doc/biovizion_mem oir_
touchette_en.pdf [retrieved on 2011-08-11]**
• **ANDREA POLESEL ET AL: "Image Enhancement
via Adaptive Unsharp Masking", IEEE
TRANSACTIONS ON IMAGE PROCESSING, IEEE
SERVICE CENTER, PISCATAWAY, NJ, US, vol. 9,
no. 3, 1 March 2000 (2000-03-01), XP011025550,
ISSN: 1057-7149**

**Description**

TECHNICAL FIELD

The present invention relates to a surface analysis method and system. More specifically, the present invention relates to a surface analysis method and system for the diagnostic of postural abnormalities in the structure of the human body.

BACKGROUND

Various non-invasive biomedical investigation and diagnosis methods and systems have been explored, in particular optical methods and systems because of the relative simplicity and affordability of the equipment employed.
Different known optical methods involve both passive and active means of optically investigating the organism. In the first case, the organism's own radiation at the infrared (IR) range is recorded, while in the second case, external illumination of insignificant density, absolutely harmless for the human organism, is employed.
In the case of IR investigation, the recorded thermal radiation results from the metabolic generation of heat emanating from the human body. The patterns of such thermal emissions are affected by the activities of the tissues, organs and vessels inside the body. The amount of radiation can reflect the metabolic rate of the human body.
For example, US patent No. 6,023,637 entitled "Method and apparatus for thermal radiation imaging", issued to Liu *et al.* on February 8, 2002, discloses a method and apparatus for obtaining images reflecting the metabolic activity within the body of a patient. This is accomplished using digital images indicative of the patient's body IR intensity. The various IR intensities are assigned distinct colors, which forms a new image reflecting the patient's metabolic activity.
In the case of external illumination, one of the most common applications is the investigation of skin condition. For example, angled lighting has been used to generate a gradient of the illuminating field on the skin in order to enhance the visualization of wrinkles and fine lines. Depending on the direction of the gradient (vertical or horizontal), different sets of wrinkles and fine lines may be visually enhanced.
Polarized light photography has also been developed to selectively enhance either surface or subsurface features of the skin. These results are accomplished by placing a polarizing filter (typically a linear polarizing filter) both in front of the flash unit, and in front of the camera. When the polarizing filters are in the same orientation with each other, surface features of the skin such as scales, wrinkles, fine lines, pores, and hairs are visually enhanced. When the polarizing filters are aligned perpendicular to each other, subsurface features of the skin such as erythema, pigmentation and blood vessels are visually enhanced.
Ultraviolet photography, where the flash unit is filtered to produce ultraviolet A (UVA) light and the camera is filtered so that only visible light enters the lens, has been used to visually enhance the appearance of pigmentation, the bacteria p. acnes, and horns. A variation of ultraviolet photography has been termed the "sun camera" where UVA light is used to illuminate the skin and an UVA sensitive film or a digital camera is used to record the reflected ultraviolet light from the skin. In this arrangement, both the pigment distribution and the surface features of the skin are visually enhanced.
For example, US Patent No. 6,907,193, entitled "Method of taking polarized images of the skin and the use thereof", issued to Kollias *et al.* on June 14, 2006, discloses a method of investigation of the skin using first a white light, followed by an ultraviolet light and finally a phosphorescent blue light. Each time a specific lighting is used, a picture of the patient is taken at an angle between 35 and 55 degrees. The angle allows the amplification of skin characteristics such as fine lines, skin texture, hairs, etc. Furthermore, the use of filters, such as polarizing filters is described. High frequency filters, red light blocking filters, etc. are also used to amplify some characteristics of the skin.
Another example is US patent No. 5,747,789, entitled "Method for investigation of distribution of physiological components in human body tissues and apparatus for its realization", issued to Godik on May 5th 1998, which discloses a method for the investigation a region of a patient's body. The method begins by illuminating the region under investigation and recording, at regular intervals, the spatial distribution of the intensity of the reflected light using, for example, a digital camera. The sequence of spatial distribution of the intensity of the reflected light thus obtained gives information on a spatial picture of the functional dynamics of the arterial and venous capillary blood content. Depending on the physiological component to be investigated, a light source composed of specific wavelengths is used in order to heighten the sensitivity of the method. This wavelength specific light source is produced with the use of optical filters.
Finally, US patent application No. 2004/0125996, entitled "Skin diagnostic imaging method and apparatus", naming Eddowes *et al.* as inventors and published on July 1st 2004, discloses a method and apparatus for face skin diagnostic, the method consisting in illuminating the face of a patient with a white light combined with red and blue or red and green filters, and taking a digital images of the patient's face thus illuminated. A digital image of the patient's face is also taken using an ultraviolet light source. The images thus obtained are analyzed by a computer program which identifies skin regions requiring preventive skin treatment.
Elodie Touchette: "Identification of the morphological parameters characteristic of pes cavus and the development of a prediction model", 1 June 2003 (2003-06-01), XP55004607, (http://www.cryos.com/doc/biovizion_memoir_ touchette_en.pdf), discloses a system for distinguishing pathological feet from healthy feet by identifying angles characteristic associated with the pes cavus pathology. The system uses a camera to take digital pictures of the patient's feet placed in different positions. The digital pictures are then processed with a graphic filter and used to measure the characteristic angles of the patient's foot morphology in order to determine the presence or not of the pes cavus pathology.

Andrea Polesel et al: "Image Enhancement via Adaptive Unsharp Masking", IEEE TRANSACTIONS ON IMAGE PROCESSING, IEEE SERVICE CENTER, PISCATAWAY, NJ, US, vol. 9, no. 3, 1 March 2000 (2000-03-01), XP011 025550, ISSN: 1057-7149, discloses a method for unsharp masking for contrast enhancement of images employing an adaptive filter that controls the contribution of the sharpening path in such a way that contrast enhancement occurs in high detail areas and little or no image sharpening occurs in smooth areas.

US 5 682 443 A discloses a method and apparatus relating to the real time automatic detection and classification of characteristic type surface imperfections occurring on the surfaces of material of interest such as moving hot metal slabs produced by a continuous steel caster. A data camera transversely scans continuous lines of such a surface to sense light intensities of scanned pixels and generates corresponding voltage values. The voltage values are converted to corresponding digital values to form a digital image of the surface, which is subsequently processed to form an edge-enhanced image having scan lines characterized by intervals corresponding to the edges of the image. The edge-enhanced image is thresholded to segment out the edges and objects formed by the edges are segmented out by interval matching and bin tracking. Features of the objects are derived and such features are utilized to classify the objects into characteristic type surface imperfections.

US 6, 697,516 A discloses a method for inspecting a surface of a moving strip to detect surface defects, which includes forming a digital picture of at least one face of the strip, in which the digital picture is made up of a set of successive rows of picture elements. The method also includes filtering one digital picture to detect surface irregularities by detecting relative variations in the digital values.

WO 2005065293 A discloses a polarized material inspection apparatus that includes a light source, a first polarizing filter disposed within the optical path of the light source, a frame into which a second polarizing filter is disposed, and a support for positioning the frame such that an object may be viewed through the second polarizing filter.

There is a need for a simple non-invasive analysis method and system, which does not require sophisticated equipment, for the diagnostic of postural abnormalities in the structure of the human body.

SUMMARY

The present invention relates to a surface analysis system according to claim 1 and a surface analysis method according to claim 6.

BRIEF DESCRIPTION OF THE DRAWINGS

A non-limitative illustrative embodiment of the invention will now be described by way of example only with reference to the accompanying drawings, in which:

Figure 1 is a flow diagram of a surface analysis method according to a non-limitative illustrative embodiment of the present invention;

Figure 2 is a digital image of the front view of a patient's feet;

Figure 3 is the digital image of Figure 2 to which an inverse filter was applied;

Figure 4 is a flow diagram of an inverse filter algorithm;

Figure 5 is the digital image of Figure 2 to which a solarize filter with a level equal to 0 was applied;

Figure 6 is the digital image of Figure 2 to which a solarize filter with a level equal to 128 was applied;

Figure 7 is a flow diagram of a solarize filter algorithm;

Figure 8 is the digital image of Figure 2 to which an edge detect and inverse filters were applied;

Figures 9a and 9b is a flow diagram of an edge detect filter algorithm;

Figure 10 is the digital image of Figure 2 to which a custom filter was applied;

Figure 11 is the digital image of Figure 2 to which the custom and inverse filters were applied;

Figures 12a and 12b is a flow diagram of the custom filter algorithm;

Figure 13 is a digital image of the back of a patient to which was applied the custom filter with a level of 255 followed by the inverse filter;

Figure 14 is a digital image of the front of a patient;

Figure 15 is the digital image of Figure 14 to which was applied a custom filter with a level of 255 followed by the inverse filter;

Figure 16 is a digital image of the front view of a patient's feet, showing eversion of the lower limbs, to which was applied a custom filter with a level of 255 followed by the inverse filter;

Figure 17 is a digital image of the front view of a patient's feet, showing normal lower limbs, to which was applied the custom filter with a level of 255 followed by the inverse filter;

Figure 18 is a digital image of the back view of a patient's feet, showing eversion of the lower limbs, to which was applied the custom filter with a level of 255 followed by the inverse filter;

Figure 19 is a digital image of the back view of a patient's feet, showing normal lower limbs, to which was applied the custom filter with a level of 255 followed by the inverse filter; and

Figure 20 is a schematic view of a surface analysis system.

DETAILED DESCRIPTION

Generally stated, a method and system according to a non-limitative illustrative embodiment of the present invention provide a surface analysis system and method for the diagnostic of postural abnormalities in the structure of the human body. The method generally consist in using a digital filter, or a combination of digital filters, applied to digital images of a human body in order to highlight deformities and asymmetries on the surface of the skin covering the human body structure by accentuating the reflection of light upon the relief of the skin surface. It is to be understood that such a method may also be used in other contexts such as, for example, the analysis of the surface of the metallic body of a vehicle in order to identify any warping or indentations caused by an impact or an applied torque.

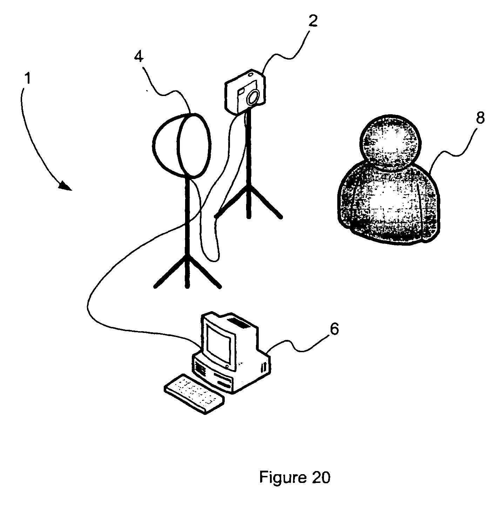

A system 1 that may be use to implement the method is shown in Figure 20 and advantageously consist of a digital camera 2, at least one flash unit, constant direct or diffuse source of light or a combination thereof 4 and a processing unit 6, such as, for example, a personal computer, to process digital images taken of a patient 8 by the digital camera 2 by applying the various filters to the digital images. It is to be understood that in an alternative embodiment the patient may be replaced by an object, for example a vehicle in the case where the surface under analysis is a metallic body of a vehicle.

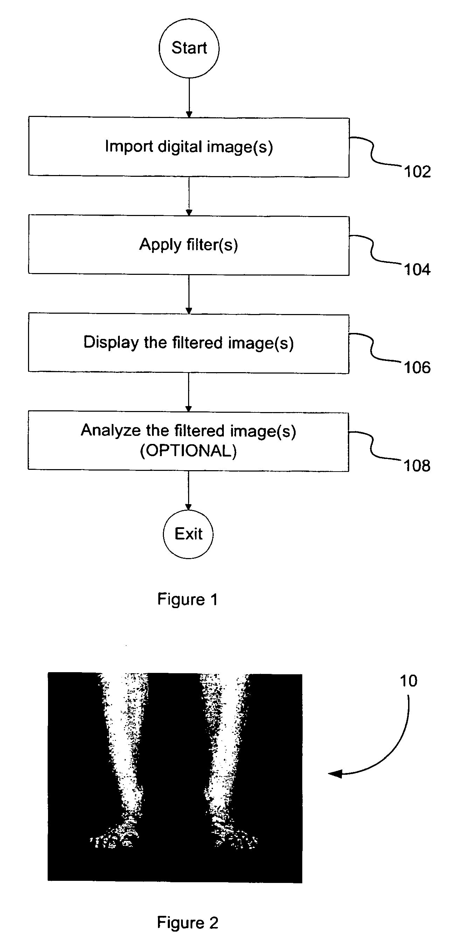

Referring to Figure 1, there is shown a flow diagram depicting the steps involved in the surface analysis method according to an illustrative embodiment of the present invention, which is indicated by blocks 102 to 106.

At block 102 the method starts by importing one, or more, digital image of the surface to be analyzed, for example a digital image of the body of a patient or the body of a vehicle. The digital image may be obtained using a digital imaging system such as, for example, a digital camera or a digital scanner or by scanning a conventional photograph or image.

Then, at block 104, one, or more, digital filter is applied to the digital image using, for example, a dedicated processor or a personal computer, in order to accentuate the reflection of light upon the surface. Four filters plus combinations of filters may be used in particular; these will be detailed further below.

At block 106, the filtered digital image are displayed, for example on a computer screen or a color printer.

Optionally, at block 108, the filtered digital image may be analyzed so as to detect deformities and asymmetries on the surface under analysis. The filtered digital image may be analyzed, for example, by a skilled technician observing the display or by an automated process recognizing certain colored structures and/or patterns.

Filters

As mentioned above, four filters and combinations of filters may be used in particular although it is to be understood that other filters or other combinations of filters may be used as well.

The first three filters are common filters, namely: the inverse filter, the solarize filter and the edge detect filter. The fourth filter is a custom type of filter. As for the combinations of filters, they are the application of the inverse filter to a digital image on which the edge detect filter has already been applied and the application of the inverse filter to a digital image on which the custom filter has already been applied.

The effects of the four filters, and the combinations of filters, will now be described with reference to Figure 2 which is an original digital image 10 of the front view of the feet of a patient.

In the following description, reference will be made to the Red Green Blue (RGB) color model, for which the intensity value of each component varies from a minimum value of 0 to a maximum value of 255. It is to be understood that other color models, having different ranges of values, may be used as well.

**Inverse filter**

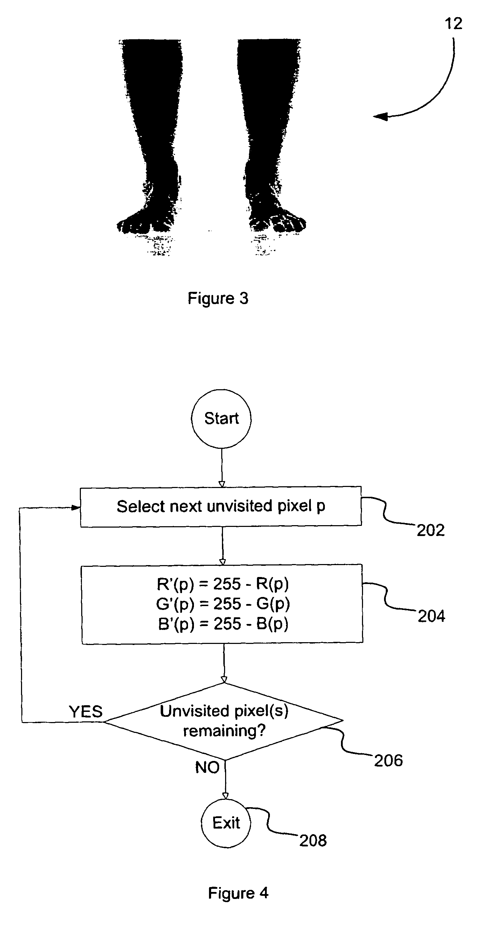

The inverse filter helps with the viewing of contrast by producing a negative image of the original digital image 10. This is achieved by inversing the intensity of the Red Green Blue (RGB) components of each pixels of the original digital image 10, i.e. the new intensity value of each of the RGB component of a given pixel will be 255 (the maximum intensity value) minus the original intensity value of that component of the pixel. For example, Figure 3 shows the inversed image 12 of the original digital image 10 of Figure 2 after the application of the inverse filter.

An illustrative example of an inverse filter algorithm that may be used is depicted by the flow diagram shown in Figure 4. The steps of the algorithm are indicated by blocks 202 to 208.

The algorithm starts at block 202 by selecting a pixel "p" of the original digital image 10 which has not yet been selected. At block 204, new intensity values of the RGB components are computed for pixel p using the following equations:

$$R'(p) = 255 - R(p); \qquad\qquad\qquad \text{Equation 1}$$

$$G'(p) = 255 - G(p); \qquad\qquad\qquad \text{Equation 2}$$

$$B'(p) = 255 - B(p); \qquad\qquad\qquad \text{Equation 3}$$

where

R'(p) is the new red component intensity value of pixel p after the application of the inverse filter, R(p) being the original red component intensity value of pixel p;

G'(p) is the new green component intensity value of pixel p after the application of the inverse filter, G(p) being the original green component intensity value of pixel p; and

B'(p) is the new blue component intensity value of pixel p after the application of the inverse filter, B(p) being the original blue component intensity value of pixel p.

Then, at block 206, the algorithm verifies if there are any remaining pixels that have not yet been selected, if so it returns to block 202, if not it exits at block 208.

**Solarize filter**

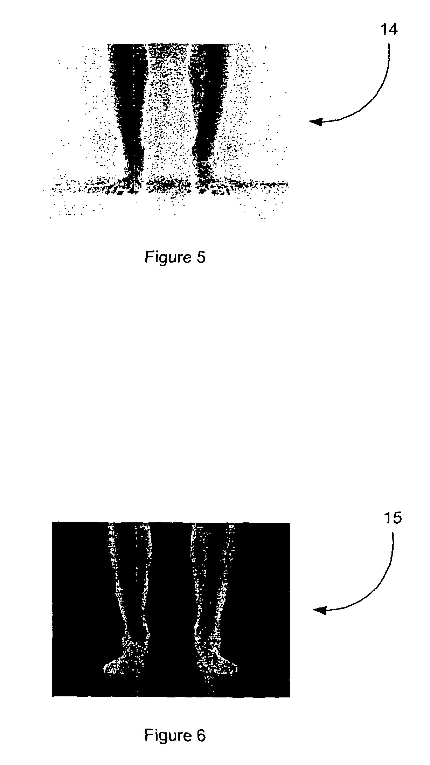

The solarize filter is similar in concept to the inverse filter with the difference that, for each pixel, the solarize filter only inverses the intensity value of the RGB components which are smaller or equal to a predetermined level "L", the level having a value in between 0 (the minimum intensity value) and 255 (the maximum intensity value). Basically, the solarize filter may be used to invert the RGB intensity values for low intensity pixels of a digital image. Thus, if the level is set at 255, the solarize filter's effect is the same as that of the inverse filter. Figures 5 and 6 show examples of effects of the solarize filter upon the original digital image 10 of Figure 2. In Figure 5 the level is set to 0, resulting in digital image 14, while in Figure 6 the level is set to 128, resulting in digital image 15.

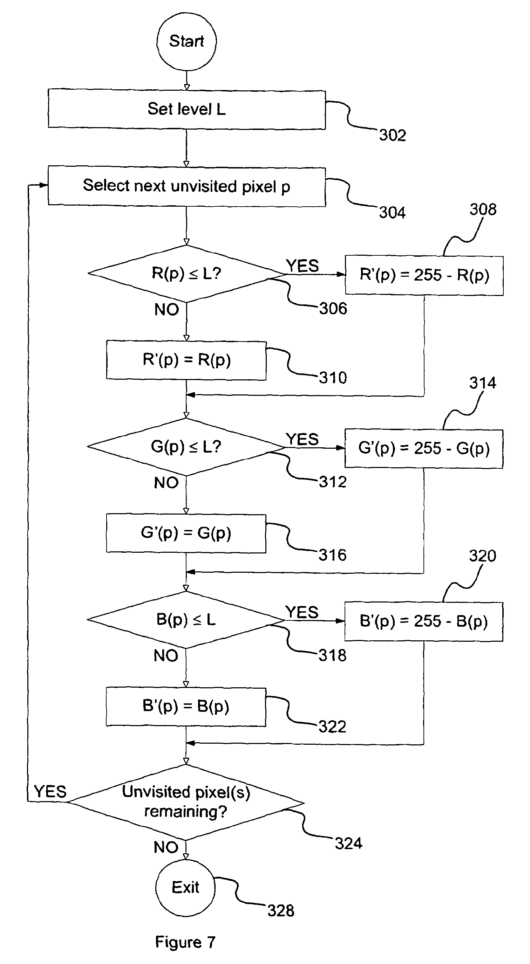

An illustrative example of a solarize filter algorithm that may be used is depicted by the flow diagram shown in Figure 7. The steps of the algorithm are indicated by blocks 302 to 328.

The algorithm starts at block 302 by setting the level L and then, at block 304, selecting a pixel "p" of the original digital image 10 which has not yet been selected. At block 306, the red component intensity value of pixel p, R(p), is compared with level L, if R(p) is lower than L, then the algorithm proceeds to block 308 and computes the new red component intensity value of pixel p using Equation 1, if not, the algorithm proceeds to block 310 where the new red component intensity value of pixel p is computed using the following equation:

$$R'(p) = R(p). \qquad\qquad\qquad \text{Equation 4}$$

At block 312, the green component intensity value of pixel p, G(p), is compared with level L, if G(p) is lower than L, then the algorithm proceeds to block 314 and computes the new green component intensity value of pixel p using Equation 2, if not, the algorithm proceeds to block 316 where the new green component intensity value of pixel p is computed using the following equation:

$$G'(p) = G(p). \qquad\qquad\qquad \text{Equation 5}$$

Similarly, At block 318, the blue component intensity value of pixel p, B(p), is compared with level L, if B(p) is lower than L, then the algorithm proceeds to block 320 and computes the new blue component intensity value of pixel p using Equation 3, if not, the algorithm proceeds to block 322 where the new blue component intensity value of pixel p is computed using the following equation:

$$B'(p) = B(p). \hspace{4cm} \text{Equation 6}$$

Then, at block 324, the algorithm verifies if there are any remaining pixels that have not yet been selected, if so it returns to block 302, if not is exits at block 328.

**Edge detect filter**

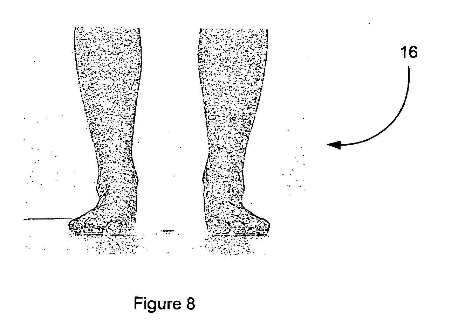

The purpose of the edge detect filter is to highlight edges between high intensity and low intensity areas of the original digital image 10, i.e. the limit between areas having high RGB intensity variations. For each RGB component intensity value of a given pixel, the value of the difference between the intensity value of that RGB component and the average of the intensity values of the eight (8) neighboring pixels, for that same RGB component, is computed. If that difference value is greater than a certain level "L", then it is set to 255 (the maximum intensity value). Finally, the pixel's new three RGB intensity values are set to the value of the RGB component having the greatest difference value. This results in a shades of grey image where the lighter lines identify edges and contours in the original digital image 10. For example, Figure 8 shows the resulting image 16 after the application of the edge detect filter and the inverse filter to the original digital image 10 of Figure 2. The inverse filter simply being applied for added clarity in order to show the edge lines in dark lines over a light background instead of light lines on a dark background.

It is to be understood that the background of the original digital image 10 may be selected according to the surface being photographed so as to provide improved contrast.

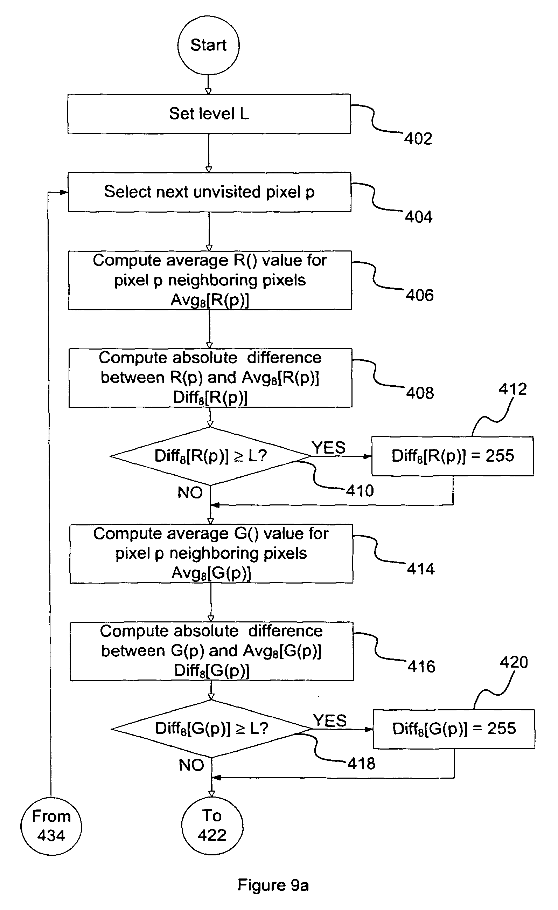

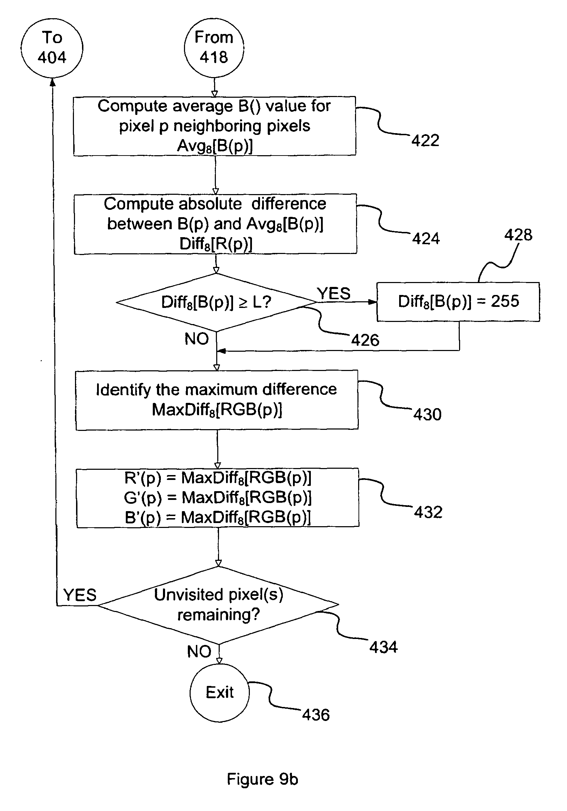

An illustrative example of an edge detect filter algorithm that may be used is depicted by the flow diagram shown in Figures 9a and 9b. The steps of the algorithm are indicated by blocks 402 to 436.

The algorithm starts at block 402 by setting the level L and then, at block 404, selecting a pixel "p" of the original digital image 10 which has not yet been selected. At block 406, the average of the red component intensity values of the eight (8) neighboring pixels to pixel p, $Avg_8[R(p)]$, is computed using the following equation:

$$Avg_8[R(p)] = Avg_8[R(x,y)] = \frac{1}{8}\left[\sum_{i=-1}^{+1}\sum_{j=-1}^{+1,j\neq i}R(x+i, y+j)\right]; \quad \text{Equation 7}$$

where
x and y are the coordinates of pixel p.

Following which, at block 408, the absolute difference between the red component intensity value of pixel p, R(p), and the average $Avg_8[R(p)]$ of block 406 is computed as $Diff_8[R(p)]$. More specifically:

$$Diff_8[R(p)] = |\ R(p) - Avg_8[R(p)]\ |. \hspace{3cm} \text{Equation 8}$$

Then, at block 410, $Diff_8[R(p)]$ is compared with level L, if $Diff_8[R(p)]$ is greater than L, then the algorithm proceeds to block 412 where it sets $Diff_8[R(p)]$ to 255 and then proceeds to block 414, if not, the algorithm proceeds to block 414.

At block 414, the average of the green component intensity values of the eight (8) neighboring pixels to pixel p, $Avg_8[G(p)]$, is computed using the following equation:

$$Avg_8[G(p)] = Avg_8[G(x,y)] = \frac{1}{8}\left[\sum_{i=-1}^{+1}\sum_{j=-1}^{+1,j\neq i} G(x+i, y+j)\right]; \quad \text{Equation 9}$$

where

x and y are the coordinates of pixel p.

Following which, at block 416, the absolute difference between the green component intensity value of pixel p, G(p), and the average $Avg_8[G(p)]$ of block 414 is computed as $Diff_8[G(p)]$. More specifically:

$$Diff_8[G(p)] = |\,G(p) - Avg_8[G(p)]\,|. \qquad \text{Equation 10}$$

Then, at block 418, $Diff_8[G(p)]$ is compared with level L, if $Diff_8[G(p)]$ is greater than L, then the algorithm proceeds to block 420 where it sets $Diff_8[G(p)]$ to 255 (the maximum intensity value) and then proceeds to block 422, if not, the algorithm proceeds to block 422.

At block 422, the average of the blue component intensity values of the eight (8) neighboring pixels to pixel p, $Avg_8[B(p)]$, is computed using the following equation:

$$Avg_8[B(p)] = Avg_8[B(x,y)] = \frac{1}{8}\left[\sum_{i=-1}^{+1}\sum_{j=-1}^{+1,j\neq i} B(x+i, y+j)\right]; \quad \text{Equation 11}$$

where

x and y are the coordinates of pixel p.

Following which, at block 424, the absolute difference between the green component intensity value of pixel p, G(p), and the average $Avg_8[G(p)]$ of block 422 is computed as $Diff_8[B(p)]$. More specifically:

$$Diff_8[B(p)] = |\,B(p) - Avg_8[B(p)]\,|. \qquad \text{Equation 12}$$

Then, at block 426, $Diff_8[B(p)]$ is compared with level L, if $Diff_8[B(p)]$ is greater than L, then the algorithm proceeds to block 428 where it sets $Diff_8[B(p)]$ to 255 (the maximum intensity value) and then proceeds to block 430, if not, the algorithm proceeds to block 430.

At block 430, the algorithm identifies the maximum absolute difference $MaxDiff_8[RGB(p)]$ among $Diff_8[R(p)]$, $Diff_8[G(p)]$ and $Diff_8[B(p)]$, and, at block 432, assigns $MaxDiff_8[RGB(p)]$ to each new individual RGB component intensity value of pixel p, i.e. R'(p), G'(p) and B'(p). Therefore, if one of the absolute differences $Diff_8[R(p)]$, $Diff_8[G(p)]$ and $Diff_8[B(p)]$ are greater than level L, all the individual RGB component intensity value of pixel p will be set to 255 (the maximum intensity value).

Then, at block 434, the algorithm verifies if there are any remaining pixels that have not yet been selected, if so it returns to block 404, if not is exits at block 436.

**Custom filter**

For each pixel the custom filter applies two different sets of rules, one for the green and blue components and one for the red component. It is to be understood that in the illustrative embodiment the red component is particularly present in the skin of a patient, in other applications it may be the green or the blue components which may warrant a different rule.

For the green and blue components of a given pixel "p", a value equal to the product of the component's intensity value and a predetermined levels "$L_G$" or "$L_B$" divided by 100 is added to that component's original intensity value to yield the resulting component intensity value. It is to be understood that any resulting intensity value lower than the minimum value, in this case 0, is set to 0 (possible in the case where $L_G$ or $L_B$ has a negative value) and that any resulting intensity value greater than the maximum value, in this case 255, is set to 255.

For the red component of the given pixel, a value equal to the product of the red intensity value, to which is subtracted the value of the red component intensity values of the eight (8) neighbouring pixels, and a predetermined level "$L_R$" divided by 100 is added to the red component's original intensity value to yield the resulting red intensity value.

Again, it is to be understood that any resulting intensity value lower than 0 is set to 0 and that any resulting intensity value greater than 255 is set to 255.

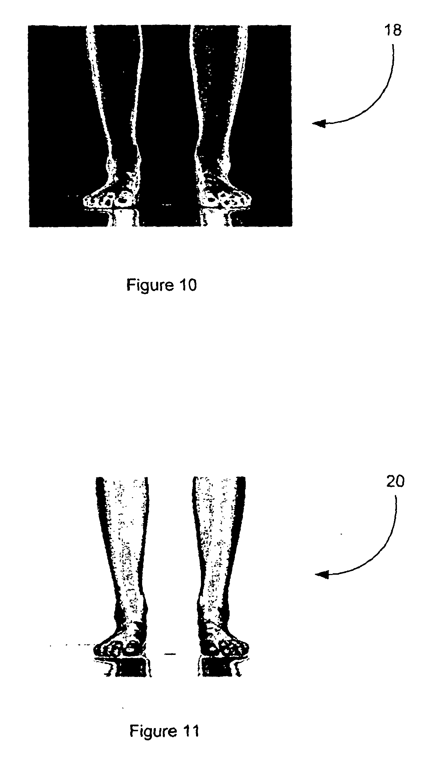

For example, Figure 10 shows the resulting image 18 after the application of the custom filter to the original digital image 10 of Figure 2. As for Figure 11, it shows the resulting image 20 after the application of both the custom filter and the inverse filter to the original digital image 10 of Figure 2, the inverse filter simply being applied for added clarity.

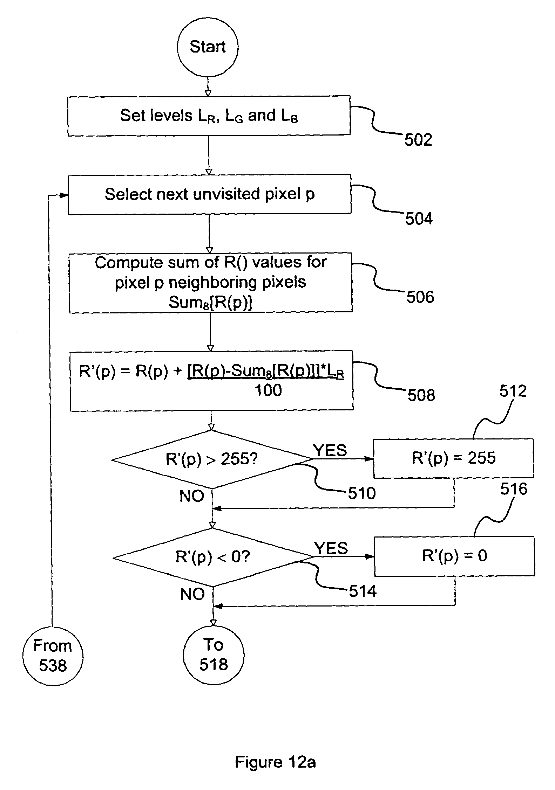

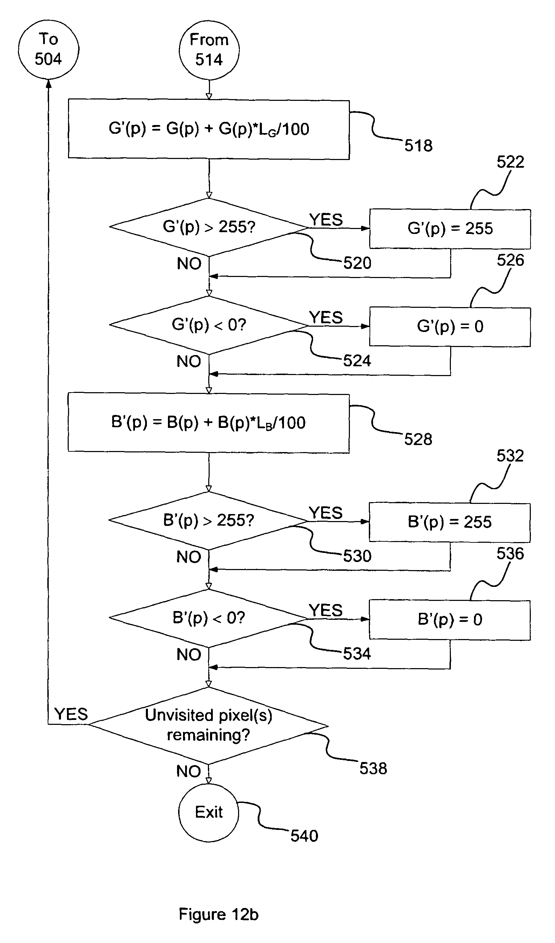

An illustrative example of the custom filter algorithm that may be used is depicted by the flow diagram shown in Figures 12a and 12b. The steps of the algorithm are indicated by blocks 502 to 540.

The algorithm starts at block 502 by setting the levels $L_R$, $L_G$ and $L_B$, and then, at block 504, selecting a pixel "p" of the original digital image 10 which has not yet been selected. At block 506, the sum of the red component intensity values of the eight (8) neighboring pixels to pixel p, $Sum_8[R(p)]$, is computed using the following equation:

$$Sum_8[R(p)] = Sum_8[R(x,y)] = \sum_{i=-1}^{+1} \sum_{j=-1}^{+1, j \neq i} R(x+i, y+j); \qquad \text{Equation 13}$$

where
x and y are the coordinates of pixel p.

Following which, at block 508, the new value of the red component intensity value of pixel p, R'(p), is computed using the following equation:

$$R'(p) = R(p) + \frac{[R(p) - Sum_8[R(p)]] \cdot L_R}{100}. \qquad \text{Equation 14}$$

Then, at block 510, the algorithm verifies if R'(p) is greater than 255 (the maximum intensity value), if so then the algorithm proceeds to block 512 where it sets R'(p) to 255, if not, the algorithm proceeds to block 514 where it verifies if R'(p) is lower than 0 (the minimum intensity value), if so then the algorithm proceeds to block 516 where it sets R'(p) to 0.

At block 518, the new value of the green component intensity value of pixel p, G'(p), is computed using the following equation:

$$G'(p) = G(p) + \frac{G(p) \cdot L_G}{100}. \qquad \text{Equation 15}$$

Then, at block 520, the algorithm verifies if G'(p) is greater than 255 (the maximum intensity value), if so then the algorithm proceeds to block 522 where it sets G'(p) to 255, if not, the algorithm proceeds to block 524 where it verifies if G'(p) is lower than 0 (the minimum intensity value), if so then the algorithm proceeds to block 526 where it sets G'(p) to 0.

At block 528, the new value of the blue component intensity value of pixel p, B'(p), is computed using the following equation:

$$B'(p) = B(p) + \frac{B(p) \cdot L_B}{100}. \qquad \text{Equation 16}$$

Then, at block 530, the algorithm verifies if B'(p) is greater than 255 (the maximum intensity value), if so then the algorithm proceeds to block 532 where it sets B'(p) to 255, if not, the algorithm proceeds to block 534 where it verifies if B'(p) is lower than 0 (the minimum intensity value), if so then the algorithm proceeds to block 536 where it sets G'(p) to 0.

Then, at block 538, the algorithm verifies if there are any remaining pixels that have not yet been selected, if so it returns to block 504, if not is exits at block 540.

**Filter combinations**

As mentioned previously, the various filters may be used individually or in combination. For example, Figure 8 illustrates the combination of the edge detect filter with the inverse filter while Figure 11 illustrates the combination of the custom filter with the inverse filter.

**Analysis**

In the illustrative embodiment described herein, the surface analysis method is used in the context of the diagnostic of postural abnormalities in the structure of the human body. The patient's postural evaluation is based on the detection of light reflection pattern changes on the surface of his or her skin. Those changes are influenced by the position of the patient's different body segments compared to each other and by muscle mass and/or tension differences.

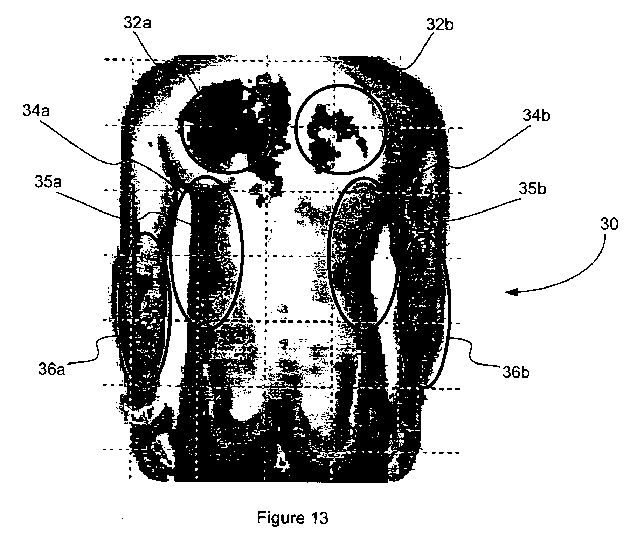

Referring to Figure 13, there is shown a treated image 30 of the back of a patient after the application of the custom filter with levels "$L_R$", "$L_G$" and "$L_B$" of 255 followed by the inverse filter. It may be seen that the light reflection patterns on the left side of the patient, more particularly in areas 32a, 34a and 36a, differ from those on the right side, that is areas 32b, 34b and 36b. It may be observed that there is less light reflected off the right scapula area 32b compared to the left scapula area 32a. From this it may be deduced that the right scapula area 32b is further away from the camera, indicating a possible postural problem. The treated image 30 also permits the identification of abnormalities of the underlying muscle structure on the right side of the patient, by comparing lines 35a and 35b.

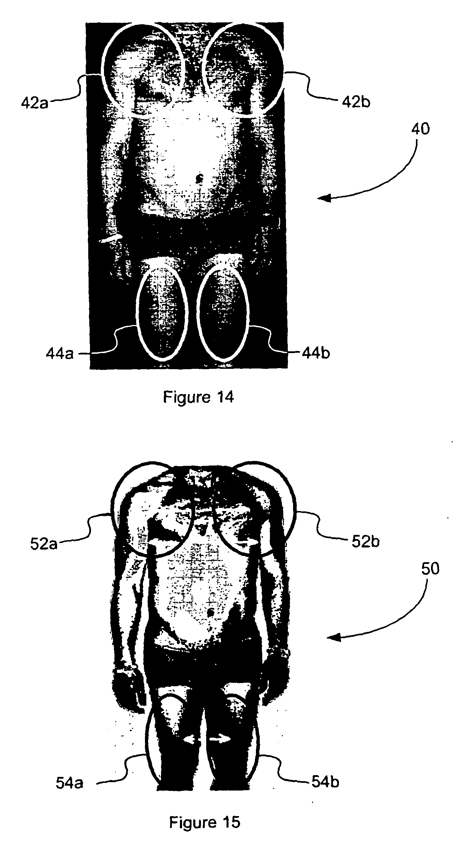

Referring now to Figures 14 and 15, there is shown an untreated digital image 40 of a patient (Figure 14) and the resulting treated image 50 (Figure 15) after the application of the custom filter with levels "$L_R$", "$L_G$" and "$L_B$" of 255 followed by the inverse filter. Referring to Figure 14, when observing the right and left shoulder areas, 42a and 42b, respectively, and the right and left upper leg areas, 44a and 44b, respectively, no obvious abnormalities or asymmetries may be easily observed. Referring now to Figure 15, the treated image 50 now shows clear abnormalities and asymmetries in the same corresponding areas, namely right and left shoulders 52a, 52b and right and left upper legs 54a, 54b, which may help a practitioner in establishing a diagnostic.

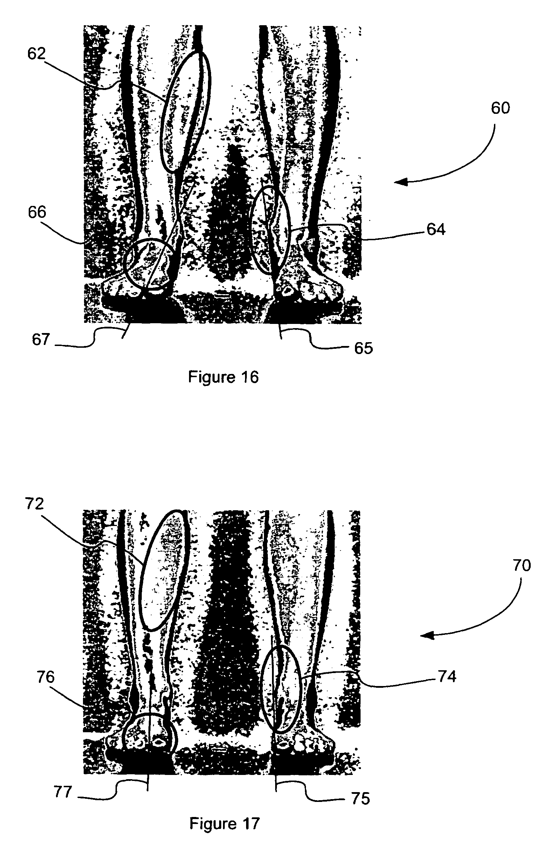

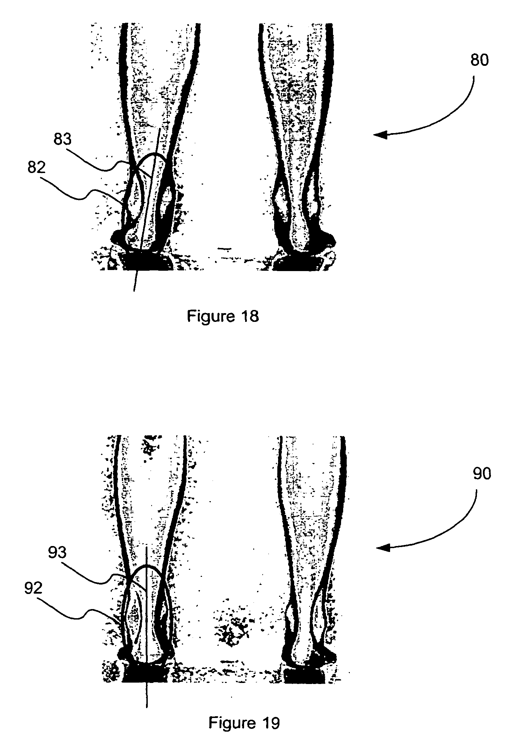

An example of the application of the custom filter, with levels "$L_R$", "$L_G$" and "$L_B$" of 255, followed by the inverse filter to a digital image of a patient for the diagnostic of a physiological condition is illustrated in Figures 16 to 19. Figures 16 and 17 show front views of the lower limbs of two differrent patients while Figures 18 and 19 respectively show back views of the lower limbs of the same patients.

Referring to Figure 16, it may be seen that the treated image 60 shows signs of eversion of the lower limbs while the treated image 70 of Figure 17 shows normal lower limbs. This may be deduced by various factors, such as, for example, observing that the calf 62 of treated image 60 is less illuminated and the reflection less uniform than that the calf 72 of treated image 70, which is an indicator of the presence of tibial rotation.

Another sign of eversion may be seen by examining the ankle regions 64 and 74, and observing that in treated image 60 the intern malleoli and the navicular bone, illustrated by line 65, are medially positionned compared to normal, which is illustrated by line 75 on treated image 70. A further sign of eversion may be seen by examining the foot region 66 of treated image 60 and tracing a line 67 in the center of the brightest portion of the light reflection, indicating the direction of the foot's center of gravity. As it may be observed, line 67 is at an angle with the vertical, this is an indication that the foot's center of gravity is not centered. Conversely, examining the foot region 76 of treated image 70 and tracing a line 77 in the center of the brightest portion of the light reflection, it may be observed that line 77 is vertical, indicating that the foot's center of gravity is in the middle of the foot and thus normal.

Referring now to Figure 18, it may be seen that the treated image 80 also shows signs of eversion of the lower limbs while the treated image 90 of Figure 19 shows normal lower limbs. An indication of eversion may be seen by examining the heel region 82 of treated image 80 and tracing a line 83 in the center of the brightest portion of the light reflection, indicating the alignment of the Achillies tendon. As it may be observed, line 83 is at an angle with the vertical, this is an indication that the Achillies tendon is inclined. Conversely, examining the heel region 92 of treated image 90 and tracing a line 93 in the center of the brightest portion of the light reflection, it may be observed that line 93 is vertical, indicating that the Achillies tendon is straight and thus normal.

Treated images such as those shown above may be taken before each treatment given to a patient in order to observe the progress of the treatment and, if necessary, readjust it.

It is to be understood that a physician or other skilled professional may use other reference structures highlighted by the treated image in order to help him or her establish a diagnostic as well as compute values such as the hallux abductus angle or the Q angle which commonly require and X-ray image of the patient. It is also to be understood that other body parts or regions may be examined such as, for example, the underfoot in order to analyze the arch of the foot. It may be further understood that the above described operations may be automated using, for example, an algorithm to identify the highlighted structures and compute values such as the hallux abductus angle or the Q angle.

Although the present invention has been described by way of a non-restrictive illustrative embodiment and examples thereof, it should be noted that it will be apparent to persons skilled in the art that modifications may be applied to the present illustrative embodiment without departing from the scope of the present invention. It is also to be understood that the present invention may be used for the detection of abnormalities in other types of surfaces such as, for example, vehicle bodywork or the surfaces of high precision metal components.

**Claims**

1. A surface analysis system (1), comprising:

a digital imaging system (2) for generating a digital image of a surface (102);
a processor (6) so coupled to the digital imaging system as to receive the digital image, to process the digital image and to generate a processed digital image highlighting relief variations in the surface (104); the processor being so configured as to perform the steps of:

a. applying a first set of rules to a selected color component of each pixel of the digital image, the first set of rules comprising:

i. adding to an intensity value of the selected color component a value equal to the intensity value, from which is subtracted the sum of intensity values of eight neighboring pixels, multiplied by a predetermined fraction associated with the selected color component (508);

b. applying a second set of rules to the remaining color components of each pixel of the digital image, the second set of rules comprising:

i. adding to an intensity value of each of the remaining color components a value equal to the intensity value multiplied by a predetermined fraction associated with each of the remaining color components (518,528);

c. setting to the minimum value any color component intensity value lower than the minimum value (514, 516) (524, 526) (534,536); and
d. setting to a maximum value any color component intensity value greater than the maximum value (510, 512) (520, 522) (530, 532);

a display system (6) for displaying the processed digital image (106).

2. A surface analysis system according to claim 1, wherein the surface is a surface of a patient's body (8).

3. A surface analysis system according to claim 1, wherein the relief variations are indicative of structures underlying the surface.

4. A surface analysis system according to claim 1, wherein the processor is so configured so as to apply to the processed digital image an inverse filter (202 to 206).

5. A surface analysis system according to claim 1, wherein the selected color component is the red component.

6. A surface analysis method, comprising:

a. capturing a digital image of a surface (102);
b. processing the digital image (104) by:

b1. applying a first set of rules to a selected color component of each pixel of the digital image, the first set of rules comprising:

i. adding to an intensity value of the selected color component a value equal to the intensity value, from which is subtracted the sum of intensity values of eight neighboring pixels, multiplied by a predetermined fraction associated with the selected color component (508);

b2. applying a second set of rules to the remaining color components of each pixel of the digital image, the second set of rules comprising:

i. adding to an intensity value of each of the remaining color components a value equal to the intensity value multiplied by a predetermined fraction associated with each of the remaining color components (518, 528);

b3. setting to a minimum value any color component intensity value lower than the minimum value (514, 516) (524,526) (534,536);
b4. setting to a maximum value any color component intensity value greater than the minimum value (510, 512) (520,522) (530,532); and

c. displaying the processed digital image (106).

7.  A surface analysis method according to claim 6, wherein the surface is a surface of a patient's body (8).

8.  A surface analysis method according to claim 6, wherein the relief variations are indicative of structures underlying the surface.

9.  A surface analysis method according to claim 6, further comprising applying to the processed digital image an inverse filter (202 to 206).

10. A surface analysis method according to claim 6, wherein the selected color component is the red component.

**Patentansprüche**

1.  Ein Oberflächen-Analysesystem (1), umfassend:

ein digitales Bildgebungssystem (2) zum Generieren eines digitalen Bildes einer Oberfläche (102);
einen Prozessor (6), der mit dem digitalen Bildgebungssystem verbunden ist, um das digitale Bild zu empfangen, um das digitale Bild zu verarbeiten und ein bearbeitetes, digitales Bild zu generieren, welches Relief-Variationen auf der Oberfläche (104) betont;
wobei der Prozessor konfiguriert ist, um die Schritte auszuführen:

a. Anwenden eines ersten Satzes von Regeln auf eine ausgewählte Farbkomponente jedes Pixels des digitalen Bildes, wobei der erste Satz von Regeln umfasst:

i. Addieren eines Werts zu einem Intensitätswert der ausgewählten Farbkomponente, der gleich dem Intensitätswert ist, von dem die Summe von Intensitätswerten von acht benachbarten Pixeln, multipliziert mit einem bestimmten, mit der gewählten Farbkomponente (508) assoziierten Bruchteil, abgezogen wurde;

b. Anwenden eines zweiten Satzes von Regeln auf die verbleibenden Farbkomponenten eines jeden Pixels des digitalen Bildes, wobei der zweite Satz von Regeln umfasst:

i. Zugeben eines Werts zu einem Intensitätswert jeder der verbleibenden Farbkomponenten, der gleich dem Intensitätswert ist, multipliziert mit einem bestimmten, mit jeder der verbleibenden Farbkomponenten (518, 528) assoziierten Bruchteil;

c. Setzen jedes Intensitätswerts jeder Farbkomponente, der geringer ist als der Minimalwert (514, 516) (524, 526) (534,536), auf den Minimalwert; und
d. Setzen des Intensitätswerts jeder Farbkomponente, der größer ist als der Maximalwert (510, 512) (520, 522) (530, 532) auf einen Maximalwert;

ein Anzeigesystem (6) zum Anzeigen des verarbeiteten, digitalen Bildes (106).

2.  Oberflächen-Analysesystem nach Anspruch 1, wobei die Oberfläche eine Oberfläche des Körpers (8) eines Patienten ist.

**3.** Oberflächen-Analysesystem nach Anspruch 1, wobei Relief-Variationen für Strukturen, die unter der Oberfläche liegen, bezeichnend sind.

**4.** Oberflächen-Analysesystem nach Anspruch 1, wobei der Prozessor konfiguriert ist, um einen Umkehrfilter (202 bis 206) auf das verarbeitete, digitale Bild anzuwenden.

**5.** Oberflächen-Analysesystem nach Anspruch 1, wobei die ausgewählte Farbkomponente die rote Komponente ist.

**6.** Oberflächen-Analyseverfahren, umfassend:

a. Aufnehmen eines digitalen Bildes einer Oberfläche (102);
b. Verarbeiten des digitalen Bildes (104) durch:

b1. Anwenden eines ersten Satzes von Regeln auf eine ausgewählte Farbkomponente jedes Pixels des digitalen Bildes, wobei der erste Satz von Regeln umfasst:

i. Addieren eines Werts zu einem Intensitätswert der ausgewählten Farbkomponente, der gleich dem Intensitätswert ist, von dem die Summe von Intensitätswerten von acht benachbarten Pixeln, multipliziert mit einem bestimmten, mit der gewählten Farbkomponente (508) assoziierten Bruchteil, abgezogen wurde;

b2. Anwenden eines zweiten Satzes von Regeln auf die verbleibenden Farbkomponenten jedes Pixels des digitalen Bildes, wobei der zweite Satz von Regeln umfasst:

i. Zugeben eines Werts zu einem Intensitätswert jeder der verbleibenden Farbkomponenten, der gleich dem Intensitätswert ist, multipliziert mit einem bestimmten, mit jeder der verbleibenden Farbkomponenten (518, 528) assoziierten Bruchteil;

b3. Setzen jedes Intensitätswerts jeder Farbkomponente, der geringer ist als ein Minimalwert (514, 516) (524, 526) (534, 536), auf einen Minimalwert; und
b4. Setzen des Intensitätswerts jeder Farbkomponente, der größer ist als der Maximalwert (510, 512) (520, 522) (530, 532) auf einen Maximalwert; und

c. Anzeigen des verarbeiteten, digitalen Bildes (106).

**7.** Oberflächen-Analyseverfahren nach Anspruch 6, wobei die Oberfläche eine Oberfläche eines Körpers (8) eines Patienten ist.

**8.** Oberflächen-Analyseverfahren nach Anspruch 6, wobei Relief-Variationen für Strukturen, die unter der Oberfläche liegen, bezeichnend sind.

**9.** Oberflächen-Analyseverfahren nach Anspruch 6, wobei der Prozessor konfiguriert ist, um einen Umkehrfilter (202 bis 206) auf das verarbeitete, digitale Bild anzuwenden.

**10.** Oberflächen-Analyseverfahren nach Anspruch 6, wobei die ausgewählte Farbkomponente die rote Komponente ist.

**Revendications**

**1.** Système d'analyse de surface (1), comprenant :

un système d'imagerie numérique (2) pour générer une image numérique d'une surface (102) ;
un processeur (6) couplé au système d'imagerie numérique de manière à recevoir l'image numérique, pour traiter l'image numérique et pour générer une image numérique traitée mettant en évidence les variations de relief de la surface (104) ; le processeur étant configuré de manière à effectuer les étapes :

a. d'application d'un premier ensemble de règles à une composante de couleur sélectionnée de chaque pixel de l'image numérique, le premier ensemble de règles comprenant :

i. l'ajout, à une valeur d'intensité de la composante de couleur sélectionnée, d'une valeur égale à la valeur d'intensité, de laquelle est soustraite la somme des valeurs d'intensité de huit pixels voisins, multipliée par une fraction prédéterminée associée à la composante de couleur sélectionnée (508) ;

b. d'application d'un deuxième ensemble de règles aux composantes de couleur restantes de chaque pixel de l'image numérique, le deuxième ensemble de règles comprenant :

i. l'ajout, à une valeur d'intensité de chacune des composantes de couleur restantes, d'une valeur égale à la valeur d'intensité multipliée par une fraction prédéterminée associée à chacune des composantes de couleur restantes (518, 528) ;

c. d'initialisation à la valeur minimum de toutes les valeurs d'intensité de composante de couleur inférieures à la valeur minimum (514, 516) (524, 526) (534, 536) ; et
d. d'initialisation à une valeur maximum de toutes les valeurs d'intensité de composante de couleur supérieures à la valeur maximum (510, 512) (520, 522) (530, 532) ;

un système d'affichage (6) pour afficher l'image numérique traitée (106).

2. Système d'analyse de surface selon la revendication 1, dans lequel la surface est une surface du corps d'un patient (8).

3. Système d'analyse de surface selon la revendication 1, dans lequel les variations de relief sont indicatives des structures sous-jacentes à la surface.

4. Système d'analyse de surface selon la revendication 1, dans lequel le processeur est configuré de manière à appliquer, à l'image numérique traitée, un filtrage inverse (202 à 206).

5. Système d'analyse de surface selon la revendication 1, dans lequel la composante de couleur sélectionnée est la composante rouge.

6. Procédé d'analyse de surface, comprenant :

a. la capture d'une image numérique d'une surface (102) ;
b. le traitement de l'image numérique (104) en :

b1. appliquant un premier ensemble de règles à une composante de couleur sélectionnée de chaque pixel de l'image numérique, le premier ensemble de règles comprenant :

i. l'ajout, à une valeur d'intensité de la composante de couleur sélectionnée, d'une valeur égale à la valeur d'intensité, de laquelle est soustraite la somme des valeurs d'intensité de huit pixels voisins, multipliée par une fraction prédéterminée associée à la composante de couleur sélectionnée (508) ;

b2. appliquant un deuxième ensemble de règles aux composantes de couleur restantes de chaque pixel de l'image numérique, le deuxième ensemble de règles comprenant :

i. l'ajout, à une valeur d'intensité de chacune des composantes de couleur restantes, d'une valeur égale à la valeur d'intensité multipliée par une fraction prédéterminée associée à chacune des composantes de couleur restantes (518, 528) ;

b3. initialisant à une valeur minimum toutes les valeurs d'intensité de composante de couleur inférieures à la valeur minimum (514, 516) (524, 526) (534, 536) ;
b4. initialisant à une valeur maximum toutes les valeurs d'intensité de composante de couleur supérieures à la valeur maximum (510, 512) (520, 522) (530, 532); et

c. l'affichage de l'image numérique traitée (106).

**7.** Procédé d'analyse de surface selon la revendication 6, dans lequel la surface est une surface du corps d'un patient (8).

**8.** Procédé d'analyse de surface selon la revendication 6, dans lequel les variations de relief sont indicatives des structures sous-jacentes à la surface.

**9.** Procédé d'analyse de surface selon la revendication 6, comprenant en outre l'application, à l'image numérique traitée, d'un filtrage inverse (202 à 206).

**10.** Procédé d'analyse de surface selon la revendication 6, dans lequel la composante de couleur sélectionnée est la composante rouge.

Figure 1

Figure 2

Figure 3

Figure 4

Figure 5

Figure 6

Figure 7

Figure 8

Figure 9a

To
404

From
418

Compute average B() value for
pixel p neighboring pixels
Avg$_8$[B(p)]
422

Compute absolute difference
between B(p) and Avg$_8$[B(p)]
Diff$_8$[R(p)]
424

428

Diff$_8$[B(p)] ≥ L? —YES→ Diff$_8$[B(p)] = 255

NO

426

Identify the maximum difference
MaxDiff$_8$[RGB(p)]
430

R'(p) = MaxDiff$_8$[RGB(p)]
G'(p) = MaxDiff$_8$[RGB(p)]
B'(p) = MaxDiff$_8$[RGB(p)]
432

YES — Unvisited pixel(s)
remaining?

434

NO

Exit

436

Figure 9b

Figure 10

Figure 11

Start

Set levels $L_R$, $L_G$ and $L_B$ ⟶ 502

Select next unvisited pixel p ⟶ 504

Compute sum of R() values for pixel p neighboring pixels $Sum_8[R(p)]$ ⟶ 506

$R'(p) = R(p) + \dfrac{[R(p)-Sum_8[R(p)]]*L_R}{100}$ ⟶ 508

$R'(p) > 255?$ — YES ⟶ $R'(p) = 255$   512

510

NO

516

$R'(p) < 0?$ — YES ⟶ $R'(p) = 0$

514

NO

From 538

To 518

Figure 12a

23

To
504

From
514

$$G'(p) = G(p) + G(p)^*L_G/100$$
—518

$G'(p) > 255?$ —520 | YES → | 522
$G'(p) = 255$

NO

$G'(p) < 0?$ —524 | YES → | 526
$G'(p) = 0$

NO

$$B'(p) = B(p) + B(p)^*L_B/100$$
—528

$B'(p) > 255?$ —530 | YES → | 532
$B'(p) = 255$

NO

$B'(p) < 0?$ —534 | YES → | 536
$B'(p) = 0$

NO

YES ← Unvisited pixel(s) remaining? —538

NO

Exit —540

Figure 12b

Figure 13

42a       42b

40

44a       44b

Figure 14

52a       52b

50

54a       54b

Figure 15

Figure 16

Figure 17

Figure 18

Figure 19

Figure 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6023637 A
- US 6907193 B
- US 5747789 A
- US 20040125996 A
- US 5682443 A
- US 6697516 A
- WO 2005065293 A

### Non-patent literature cited in the description

- *Identification of the morphological parameters characteristic of pes cavus and the development of a prediction model,* 01 June 2003, http://www.cryos.com/doc/biovizion_memoir_touchette_en.pdf
- Image Enhancement via Adaptive Unsharp Masking. **ANDREA POLESEL et al.** IEEE TRANSACTIONS ON IMAGE PROCESSING. IEEE SERVICE CENTER, 01 March 2000, vol. 9